# EUROPEAN PATENT APPLICATION

(11) **EP 3 875 578 A1**
(43) Date of publication of application: **08.09.2021**
(21) Application number: 19878341.7
(22) Date of filing: 30.10.2019
(51) Int. Cl.: C12N 5/071, C12N 5/0735, C12N 5/10, C12Q 1/06

(54) **METHOD FOR PRODUCING PLURIPOTENT STEM CELL HAVING RELEASED DIFFERENTIATION RESISTANCE TO MESENDODERM**

(30) Priority: 31.10.2018 JP 2018205912
(71) Applicant: Kyoto University, Kyoto-shi, Kyoto 606-8501 (JP)
(72) Inventor: ETO, Koji, Kyoto-shi, Kyoto 606-8501 (JP); YUZURIHA, Akinori, Kyoto-shi, Kyoto 606-8501 (JP)
(74) Representative: Heubeck, Christian
(86) International application number: PCT/JP2019/042608
(87) International publication number: WO 2020/090903

(57) **Abstract**

The present invention provides a method for producing a pluripotent stem cell released from resistance to differentiation into mesendodermal lineage, including subjecting a pluripotent stem cell to a treatment for activating an integrin β1-β-catenin signal transduction pathway (excluding contacting the pluripotent stem cell with laminin 421 or a fragment thereof and/or laminin 121 or a fragment thereof), and the like.

## Description

### [Technical Field]

The present invention relates to a method for producing a pluripotent stem cell released from resistance to differentiation into mesendodermal lineage. More particularly, the present invention relates to a method for producing a pluripotent stem cell released from resistance to differentiation into mesendodermal lineage, comprising subjecting a pluripotent stem cell to a treatment for activating a signal transduction pathway mediated by integrin β1 and β catenin.

### [Background Art]

Conventionally, it has been known that the easiness of differentiation of induced pluripotent stem cells (iPS cells) into blood cells (ease of differentiation) varies depending on the iPS cell line, but the reason therefor is not known. It has been desired to verify whether the cause of the difference in the ease of differentiation from iPS cells into blood cells rests in the properties of the cell line itself or the extracellular environment.

The present inventors have previously cultured pluripotent stem cells on various laminins used as extracellular matrix in feeder-free culture, and found that pluripotent stem cells maintained on laminin 511 show resistance to differentiation into a mesendodermal lineage, whereas pluripotent stem cells maintained on laminin 421 and laminin 121 are released from the differentiation resistance and tend to differentiate into a mesendodermal lineage, particularly blood cells. In addition, they have also found that the expression of genes in the downstream of the Wnt/β-catenin signal transduction pathway increases in pluripotent stem cells maintained on the above-mentioned specific laminin and released from resistance to differentiation into a mesendodermal lineage (patent document1). This finding is consistent with previous reports that activation of Wnt/β-catenin signal is important for differentiation into mesoderm and blood from ES cells and epiblasts (e.g., non-patent documents 1 - 3).

However, it remains unclear why only specific laminins can release the resistance of pluripotent stem cells to differentiation into a mesendodermal lineage.

### [Document List]

### [Patent document]

patent document 1: WO 2018/038242

### [Non-patent documents]

non-patent document 1: Liu, P. et al., Nat. Genet., 22: 361-365 (1999)
non-patent document 2: Huelsken, J. et al., J. Cell. Biol., 148: 567-578 (2000)
non-patent document 3: Woll, P.S. et al., Blood, 111(1): 122-123 (2008)

### [Summary of Invention]

### [Technical Problem]

Therefore, an object of the present invention is to elucidate the mechanism according to which a specific laminin releases the resistance of pluripotent stem cells to differentiation into a mesendodermal lineage, and provide a more efficient method for inducing differentiation from pluripotent stem cells into a mesendodermal lineage, particularly blood cells. Another object of the present invention is to provide a means for evaluating the ease of differentiation of pluripotent stem cell clones into a mesendodermal lineage by utilizing the mechanism.

### [Solution to Problem]

The present inventors considered that it is important to activate the Wnt/β-catenin signal transduction pathway in order to release resistance of pluripotent stem cells to differentiation into a mesendodermal lineage. They have found that the differentiation potential is improved and the number of hematopoietic progenitor cells increases by first maintenance-culturing pluripotent stem cells on laminin 511 at a recommended concentration in the presence of an inhibitor of GSK3β that phosphorylates β-catenin and promotes homeostatic degradation by the ubiquitin proteasome system, and thereafter inducing differentiation into hematopoietic progenitor cells.

Next, the present inventors took note of integrin β1 (ITGB1), which is a cell surface protein known as a receptor for laminin, and compared the expression levels of ITGB1 between pluripotent stem cells maintained on laminin 421 or laminin 121, which has a high differentiation induction potential into hematopoietic progenitor cells (also referred to as "hematopoietic" in the present specification) and pluripotent stem cells maintained on laminin 511 with a low differentiation induction potential into hematopoietic progenitor cells (also referred to as "non-hematopoietic" in the present specification). As a result, they have found that the former highly expressed ITGB1. It has been reported that ITGB1 changes the amount of accumulation of β-catenin via ILK/pGSK3β and regulates the Wnt/β-catenin signal transduction pathway in cancer cells. Thus, the present inventors compared the amount of ILK/pGSK3β/β-catenin between pluripotent stem cells maintained on laminin 421 or laminin 121 and pluripotent stem cells maintained on laminin 511. As a result, it was clarified that the amount of ILK/pGSK3β/β-catenin was higher in the former, and as a result, the Wnt/β-catenin signal transduction pathway was activated.

Hematopoietic laminin 421 and laminin 121 both contain β2 and γ1 as β and γ subunits. However, hematopoietic progenitor cells were not induced from pluripotent stem cells maintained on laminin 321 which also contains β2 and γ1. Thus, a mechanism other than the combination of βγ subunits was considered. Moreover, the present inventors found, in the process of maintenance culture research of pluripotent stem cells, that hematopoietic laminin 421 and laminin 121 tend to have weaker cell-matrix adhesion than non-hematopoietic laminin 511. Therefore, to examine whether the difference in the differentiation induction potential into hematopoietic progenitor cells between laminin 421 or laminin 121, and laminin 511 is due to the kind of laminin or the adhesive strength between ITGB1 and laminin, non-hematopoietic laminin 511 was serially diluted to decrease adhesive strength to ITGB1. As a result, it was clarified that the expression of ITGB1 increases more as the cell-substrate adhesion becomes weaker in pluripotent stem cells showing differentiation resistance. Furthermore, it was found that the differentiation potential is improved and the number of hematopoietic progenitor cells increases in the pluripotent stem cells maintained on the serially diluted laminin 511 and induced to differentiate into hematopoietic progenitor cells, as compared with the pluripotent stem cells maintained at the recommended concentration.

On the other hand, it was found that some pluripotent stem cell clones do not show differentiation resistance even when they are maintained on laminin 511 at the recommended concentration. It was clarified that ITGB1 is sufficiently expressed on any dilution series of laminin 511 in such pluripotent stem cells that do not show differentiation resistance. That is, it was shown that the ease of differentiation of pluripotent stem cells into a mesendodermal lineage, particularly blood cells, can be evaluated by using the expression level of ITGB1, which is a cell surface molecule, as an index, and pluripotent stem cells determined to exhibit differentiation resistance can be released from the differentiation resistance by using laminin 511 at a reduced concentration.

The present inventors have also found that the number of cells that maintain pluripotency decreases before the start of the induction of differentiation into hematopoietic progenitor cells when the Wnt/β-catenin signal transduction pathway is activated using the aforementioned GSK3β inhibitor, and that when laminin 421 or laminin 121, which has weak adhesive strength with ITGB1, is used, or when laminin 511 diluted to weaken the adhesive strength is used, the differentiation potential is improved without reducing the number of cells that maintain pluripotency. That is, by using laminin 511 at a concentration lower than the recommended concentration, both maintenance of pluripotency and release of differentiation resistance were successfully achieved simultaneously while reducing the cost necessary for the maintenance culture of the pluripotent stem cells.

The present inventor conducted further studies based on these findings and completed the present invention.

Accordingly, the present invention provides the following.
[1] A method for producing a pluripotent stem cell released from resistance to differentiation into mesendodermal lineage, comprising subjecting a pluripotent stem cell to a treatment for activating an integrin β1-β-catenin signal transduction pathway.
[2] The method of [1], wherein the aforementioned pluripotent stem cell is further released from resistance to differentiation into a mesodermal lineage.
[3] The method of [2], wherein the aforementioned pluripotent stem cell is further released from resistance to differentiation into a blood cell.
[4] The method of any of [1] to [3], wherein the aforementioned activation treatment is bringing the pluripotent stem cell into contact with laminin 511 or a fragment thereof corresponding to 0.005 - 0.25 µg/cm² of laminin 511 E8 fragment.
[5] The method of any of [1] to [3], wherein the aforementioned activation treatment is bringing the pluripotent stem cell into contact with laminin 511 or a fragment thereof corresponding to 0.03 - 0.07 µg/cm² of laminin 511 E8 fragment.
[6] The method of any of [1] to [5], wherein not less than 90% of the pluripotent stem cells are Oct3/4-positive and Sox2-positive.
[7] The method of any of [1] to [3], wherein the aforementioned activation treatment is contacting the pluripotent stem cell with a GSK3β inhibitor for 2 - 5 days.
[8] The method of any of [1] to [7], wherein the pluripotent stem cell is derived from human.
[9] A method for producing a cell of a mesodermal lineage or endodermal lineage, comprising
   (1) a step of producing a pluripotent stem cell released from resistance to differentiation into a mesendodermal lineage by the method of any of [1] to [8], and (2) a step of inducing differentiation of the cell obtained in step (1) into a cell of a mesodermal lineage or endodermal lineage.
[10] The method of [9], wherein the aforementioned cell of a mesodermal lineage is a blood cell, a vascular endothelial cell, a skeletal muscle cell, a chondrocyte, a kidney cell, a cardiomyocyte or an adipocyte.
[11] The method of [9], wherein the aforementioned cell of a mesodermal lineage is the aforementioned cell of a mesodermal lineage is a blood cell.
[12] The method of [9], wherein the aforementioned cell of an endodermal lineage is a pancreatic β cell, a hepatocyte, an intestinal cell, a lung cell or a thyroid gland.
[13] A culture kit for releasing resistance of a pluripotent stem cell to differentiation into a mesendodermal lineage, comprising a culture container coated with laminin 511 or a fragment thereof corresponding to 0.005 - 0.25 µg/cm² of laminin 511 E8 fragment.
[14] The kit of [13], wherein the aforementioned culture container is coated with laminin 511 or a fragment thereof corresponding to 0.03 - 0.07 µg/cm² of laminin 511 E8 fragment.
[15] A method for evaluating resistance of a pluripotent stem cell to differentiation into a mesendodermal lineage, comprising measuring an expression level of integrin β1 in the pluripotent stem cell.

### [Advantageous Effects of Invention]

According to the present invention, a pluripotent stem cell can be released from resistance to differentiation into a mesendodermal lineage by activating the integrin β1(ITGB1)-β-catenin signal transduction pathway. Thus, a pluripotent stem cell suitable for inducing differentiation into cells of the mesendodermal lineage can be provided. In particular, a pluripotent stem cell can be released from resistance to differentiation into a mesendodermal lineage by diluting laminin 511 or a fragment thereof to a concentration lower than the recommended concentration. Thus, the cost reduction of maintenance culture and efficient differentiation induction can be simultaneously achieved. According to the present invention, moreover, a pluripotent stem cell can be released from resistance to differentiation into a mesendodermal lineage while sufficiently maintaining the pluripotency thereof. Thus, induction of differentiation into a cell of a mesendodermal lineage can be induced more efficiently. According to the present invention, moreover, the resistance of a pluripotent stem cell to differentiation into a mesendodermal lineage can be evaluated easily using the expression level of ITGB1 as an index.

### [Brief Description of Drawings]

Fig. 1 shows that pluripotent stem cells (PSCs) maintenance-cultured on a laminin 511 E8 fragment (LM511) show resistance to differentiation into hematopoietic cells.
Fig. 2 shows that a Wnt signal agonist (GSK3β inhibitor) treatment releases the resistance of PSCs, that were cultured and maintained on LM511, to differentiation into hematopoietic progenitor cells.
Fig. 3 shows that PSCs cultured and maintained on hematopoietic laminin highly express integrin β1 (ITGB1).
Fig. 4 shows that hematopoietic laminin activates ITGB1-β-catenin signaling.
Fig. 5 shows the relationship between the LM511 concentration used for maintenance culture of PSCs (AK5) and ITGB1 expression in the PSCs.
Fig. 6 shows that PSCs (AK5) maintenance-cultured on diluted LM511 highly express ITGB1 and are efficiently induced to differentiate into hematopoietic progenitor cells.
Fig. 7 shows that regulation with a GSK3β inhibitor prevents maintenance of the pluripotency of PSCs, whereas maintenance culture of PSCs on diluted LM511 or LM421/LM121 can simultaneously achieve maintenance of pluripotency and release of differentiation resistance.
Fig. 8 shows that hematopoietic laminin 421 and laminin 121 show weaker cell-substrate adhesion than non-hematopoietic laminin 511.
Fig. 9 shows (A) differentiation potential into hematopoietic progenitor cells (HPCs), (B) relative expression level of ITGB1, and (C) correlation between the number of HPCs and the expression level of ITGB1, in three types of PSCs maintenance-cultured on the dilution series of LM511.

### [Description of Embodiments]

### (I) Production method of pluripotent stem cell released from resistance to differentiation

The present invention provides a method for releasing resistance of a pluripotent stem cell to differentiation into a mesendodermal lineage, namely, a method for producing a pluripotent stem cell released from resistance to differentiation (hereinafter to be referred to as "the production method (I) of the present invention"). Therefore, pluripotent stem cells to be subjected to the production method (I) of the present invention preferably have resistance to differentiation.

As used herein, the "resistance to differentiation" means properties that cause difficulty in differentiating a pluripotent stem cell into a desired cell when the cell is induced to differentiate into the desired cell. The "resistance to differentiation into a mesendodermal lineage" in the present invention can be shown by a difficulty in differentiating into a desired cell or a difficulty equivalent thereto or a difficulty higher than the same when, for example, human iPS cell line AK5 (Nakagawa et al., Scientific Reports, 4: 3954, 2014) is maintenance-cultured on laminin 511 or a fragment thereof at a recommended concentration (corresponding to 0.5 µg/cm² based on laminin 511 E8 fragment) and induced to differentiate into a cell of an endodermal lineage. Whether the pluripotent stem cell has a differentiation resistance can be judged by the below-mentioned "evaluation method of the present invention".

Being "released from differentiation resistance" means that the efficiency of differentiation into the desired cell significantly increases compared with that before the release treatment. Preferably, it means that the efficiency of differentiation into the desired cell increases not less than 2 times, more preferably not less than 4 times, further preferably not less than 8 times, that before the release treatment.

The "mesendodermal lineage" is used to include a cell of a mesodermal lineage, a cell of an endodermal lineage, and a mesendodermal cell which is a common precursor of the both. Therefore, to "release resistance to differentiation into a mesendodermal lineage" means to increase at least the efficiency of differentiation from a pluripotent stem cell into a mesendodermal cell than that before the release treatment. Preferably, according to the production method (I) of the present invention, a pluripotent stem cell with at least a resistance to differentiation into a mesodermal lineage, more preferably at least a resistance to differentiation into a blood cell, is provided.

The pluripotent stem cell to be used in the present invention is not particularly limited as long as it is an undifferentiated cell possessing a "self-renewal potential" that enables it to proliferate while retaining the undifferentiated state, and "differentiation pluripotency" that enables it to differentiate into all the three germ layers. Examples thereof include embryonic stem (ES) cell, iPS cell, embryonic germ (EG) cell derived from a primordial germ cell, multipotent germline stem (mGS) cell isolated in the process of establishment and culture of GS cell from testis tissue, multipotent adult progenitor cell (MAPC) isolated from bone marrow, MUSE cell and the like. The ES cell may be produced from a somatic cell by nuclear reprogramming. Preferred are ES cells or iPS cells. Pluripotent stem cell may be derived from a mammal, and preferred is a human-derived pluripotent stem cell.

ES cell can be established by removing an inner cell mass from the blastocyst of a fertilized egg of a mammal, and culturing the inner cell mass on fibroblast feeder cells. In addition, the cells can be maintained by passage culture using a culture medium added with substances such as leukemia inhibitory factor (LIF), basic fibroblast growth factor (bFGF) and the like. The methods of establishment and maintenance of human and monkey ES cells are described in, for example, USP5,843,780; Thomson JA, et al. (1995), Proc Natl. Acad. Sci. USA. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; Klimanskaya I, et al. (2006), Nature. 444:481-485 and the like.

As for human ES cell line, for example, WA01(H1) and WA09(H9) are available from WiCell Research Institute, and KhES-1, KhES-2, KhES-3 and KthES11 are available from Institute for Frontier Life and Medical Sciences, Kyoto University (Kyoto, Japan).

iPS cell is an artificial stem cell derived from a somatic cell, which can be produced by introducing a specific reprogramming factor in the form of a DNA or protein into a somatic cell, and show almost equivalent property (e.g., pluripotent differentiation and proliferation potency based on self-renewal) as ES cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO2007/069666). As used herein, the "somatic cell" means any animal cell (preferably, cells of mammals inclusive of human) excluding germ line cells and totipotent cells such as ovum, oocyte, ES cells and the like. It encompasses any of somatic cells of fetuses, somatic cells of neonates, and mature healthy or pathogenic somatic cells, and any of primary cultured cells, passage cells, and established lines of cells. Specific examples of the somatic cell include (1) tissue stem cells (somatic stem cells) such as neural stem cell, hematopoietic stem cell, mesenchymal stem cell, dental pulp stem cell and the like, (2) tissue progenitor cell, (3) differentiated cells such as lymphocyte, epithelial cell, endothelial cell, myocyte, fibroblast (skin cells etc.), hair cell, hepatocyte, gastric mucosal cell, enterocyte, splenocyte, pancreatic cell (pancreatic exocrine cell etc.), brain cell, lung cell, renal cell and adipocyte and the like, and the like.

When the iPS cells obtained are to be used for regenerative medicine in humans, it is particularly preferable, from the viewpoint of prevention of graft rejection, to collect the somatic cells from a patient or another person with the same or substantially the same HLA type as that of the patient. "Substantially the same HLA type" as used herein means that the HLA type of donor matches with that of patient to the extent that the transplanted cells, which have been obtained by inducing differentiation of iPS cells derived from the donor's somatic cells, can be engrafted when they are transplanted to the patient with use of immunosuppressant and the like. For example, it includes an HLA type wherein major HLAs (e.g., the three major loci of HLA-A, HLA-B and HLA-DR, the four loci further including HLA-C) are identical and the like.

The reprogramming factor may be constituted with a gene specifically expressed by ES cell, a gene product or non-coding RNA thereof, a gene playing an important role for the maintenance of undifferentiation of ES cell, a gene product or non-coding RNA thereof, or a low molecular weight compound. Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbxl5, ERas, ECAT15-2, Tell, beta-catenin, Lin28b, Sall1, Sall4, Esrrb, Nr5a2, Tbx3, Glis1 and the like. These reprogramming factors may be used alone or in combination.

While, from immediately after production, a pluripotent stem cell can also be maintenance-cultured after release of the resistance to differentiation into mesendodermal lineage by the production method (I) of the present invention, it can also be subjected to maintenance culture by a method known per se before the production method (I) of the present invention. Basal media for maintenance culture include, but are not limited to, Neurobasal medium, Neural Progenitor Basal medium, NS-A medium, BME medium, BGJb medium, CMRL 1066 medium, minimal essential medium (MEM), Eagle MEM, αMEM, Dulbecco's modified Eagle medium (DMEM), Glasgow MEM, Improved MEM Zinc Option medium, IMDM medium, 199 medium, DMEM/F12 medium, Ham's medium, RPMI1640 medium, Fischer's medium, and mixtures thereof. Alternatively, commercially available pluripotent stem cell medium (e.g., StemFit, AK02N, Essential 8 etc.) may also be used.

The medium can be a serum-containing or serum-free medium. Preferably, a serum-free medium can be used. The serum-free medium (SFM) refers to media with no unprocessed or unpurified serum and accordingly, can include media with purified blood-derived components or animal tissue-derived components (such as growth factors). The concentration of serum (for example, fetal bovine serum (FBS), human serum, etc.) can be 0-20%, preferably 0-5%, more preferably 0-2%, most preferably 0% (i.e., serum-free). The SFM may contain or may not contain any alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolglycerol, or equivalents thereto. The alternatives to serum can be prepared by the method disclosed in WO 98/30679, for example. Alternatively, any commercially available materials can be used for more convenience. The commercially available materials include Knockout™ Serum Replacement (KSR), Chemically-defined Lipid concentrated, and Glutamax (Invitrogen).

The medium can also contain other additives known per se. For example, growth factors (for example, insulin and the like), polyamines (for example, putrescine and the like), minerals (for example, sodium selenate and the like), saccharides (for example, glucose and the like), organic acids (for example, pyruvic acid, lactic acid and the like), amino acids (for example, non-essential amino acids (NEAA), L-glutamine and the like), reducing agents (for example, 2-mercaptoethanol and the like), vitamins (for example, ascorbic acid, d-biotin and the like), steroids (for example, [beta]-estradiol, progesterone and the like), antibiotics (for example, streptomycin, penicillin, gentamycin and the like), buffering agents (for example, HEPES and the like), nutritive additives (for example, B27 supplement, N2 supplement, StemPro-Nutrient Supplement and the like) and the like can be mentioned. It is preferable that each of the additives be contained in a concentration range known per se.

Pluripotent stem cells may be cultured in the presence or absence of feeder cells. Many of the feeder cells that can be used for culturing pluripotent stem cells, such as ESC, iPSC and the like, can by itself release the resistance of pluripotent stem cells to differentiation into a mesendodermal lineage, and often do not require the production method (I) of the present invention. In consideration of the clinical application to humans, pluripotent stem cells are desirably cultured in the absence of a feeder cell. Therefore, in a preferred embodiment of the present invention, pluripotent stem cells are cultured under feeder-free conditions.

A culture vessel used for maintenance culture of pluripotent stem cells may include, but is particularly not limited to, flask, flask for tissue culture, dish, petri dish, dish for tissue culture, multi dish, micro plate, micro-well plate, multi plate, multi-well plate, micro slide, chamber slide, schale, tube, tray, culture bag, and roller bottle. The culture vessel can be cellular adhesive. The cellular adhesive culture vessel can be coated with any of substrates for cell adhesion such as extracellular matrix (ECM) to improve the adhesiveness of the culture vessel surface to the cells. The substrate for cell adhesion can be any material intended to attach pluripotent stem cells or feeder cells (if used). The substrate for cell adhesion includes laminin, collagen, gelatin, poly-L-lysine, poly-D-lysine, poly-L-ornithine, and fibronectin and mixtures thereof for example Matrigel, and lysed cell membrane preparations (Klimanskaya I et al 2005. Lancet 365: p1636-1641). These cell adhesion substrates are coated on culture vessel at concentrations generally used for culturing pluripotent stem cells, according to the kind thereof.

In this cultivation, pluripotent stem cells are plated onto the culture vessel mentioned above to obtain a cell density of, for example, about 10⁴-10⁵ cells/cm², and can be cultured in an incubator under atmospheric conditions of 1-10% CO₂/99-90% air at about 30-40°C, preferably about 37°C.

In the step of culturing pluripotent stem cells, the medium can be changed during the culture period. The medium used for the medium exchange may be one having the same components as the medium before the medium exchange, or one having different components. Preferably, a medium having the same components is used. The time of medium exchange is not particularly limited and, for example, the medium is exchanged every 1 day, every 2 days, every 3 days, every 4 days, and every 5 days, after the start of culturing in a fresh medium.

The production method (I) of the present invention is characterized by subjecting the pluripotent stem cell to a treatment for activating an integrin β1-β-catenin signal transduction pathway. By this treatment, a pluripotent stem cell can be released from resistance to differentiation into a mesendodermal lineage.

In the present specification, the "integrin β1-β-catenin signal transduction pathway" refers to a pathway in which integrin β1 (ITGB1) activates integrin-linked kinase (ILK) upon stimulation by a binding ligand, the activated ILK phosphorylates the 9th Ser of glycogen synthase kinase 3β (GSK3β) and inactivates same, thereby suppressing the phosphorylation of β-catenin and accumulating β-catenin in the cell, as a result of which the Wnt/β-catenin signal transduction pathway is activated and the expression of its downstream gene is induced.

Examples of the treatment for activating an ITGB1-β-catenin signal transduction pathway include a treatment for increasing the expression of ITGB1 in pluripotent stem cells and/or changing ITGB1 to an activated state, and the like. Such treatment preferably includes, but is not limited to, contacting pluripotent stem cells with an ITGB1 binding ligand at a concentration capable of causing sufficient expression of ITGB1 in the pluripotent stem cells. The ITGB1 binding ligand is not particularly limited as long as it can achieve an ITGB1 expression level sufficient for activating the ITGB1-β-catenin signal transduction pathway within a specific concentration range. For example, laminin 511 or a fragment thereof is used for pluripotent stem cells having resistance to differentiation into a mesendodermal lineage, the differentiation resistance of the pluripotent stem cells can be released by contacting laminin 511 or a fragment thereof with the pluripotent stem cells at a concentration corresponding to 0.005 - 0.25 µg/cm² (1/100 - 1/2 of recommended concentration 0.5 µg/cm²), preferably 0.025 - 0.125 µg/cm² (1/20 - 1/4 of recommended concentration), more preferably 0.03 - 0.07 µg/cm² (about 1/16 - 1/7 of recommended concentration), based on laminin 511 E8 fragment. On the other hand, for pluripotent stem cells free of the "differentiation resistance" in the present specification, laminin 511 can be preferably used at a recommended concentration.

The laminin 511 fragment is not particularly limited as long as it has an adhesive strength to ITGB1 which is equivalent to that of intact laminin 511. It is desirably a fragment containing at least a region of the E8 fragment.

As for other laminins (e.g., laminin 411, laminin 311, laminin 111 and fragments thereof) and ITGB1 binding ligands other than laminin (e.g., collagen, fibronectin, E-cadherin, ADAM, etc.), a concentration that can achieve an ITGB1 expression level sufficient for activating the ITGB1-β-catenin signal transduction pathway can be appropriately selected using the expression level of ITGB1 in the pluripotent stem cell of interest as an index.

The binding ligand of ITGB1 can be contacted with pluripotent stem cells by coating the surface of the aforementioned culture vessel used for maintenance culture of the pluripotent stem cells with the binding ligand as a substrate for cell adhesion, seeding the pluripotent stem cells on the culture vessel, and performing maintenance culture in the same manner as described above. For example, when laminin 511 or a fragment thereof is used as an ITGB1 binding ligand and applied to pluripotent stem cells with differentiation resistance, a solution of laminin 511 or a fragment thereof is added to a culture vessel at a concentration corresponding to 0.005 - 0.25 µg/cm² (1/100 - 1/2 of recommended concentration 0.5 µg/cm²), preferably 0.025 - 0.125 µg/cm² (1/20 - 1/4 of recommended concentration), more preferably 0.03 - 0.07 µg/cm² (about 1/16 - 1/7 of recommended concentration), based on laminin 511 E8 fragment, and the mixture is stood for a given time, whereby laminin 511 or a fragment thereof can be coated on the surface of the culture vessel.

The contact time between the ITGB1 binding ligand and pluripotent stem cells is not particularly limited as long as it is sufficient for activating the ITGB1-β-catenin signal transduction pathway and is, for example, not less than 2 days, preferably not less than 3 days. When the ITGB1-β-catenin signal transduction pathway is activated by contact with the ITGB1 binding ligand, since the number of cells that retain pluripotency in the pluripotent stem cell population is sufficiently maintained (Oct3/4-positive and Sox2-positive cells are 90%, preferably not less than 95%, of the entirety) during the contact, i.e., throughout the culture period in a culture vessel coated with the ligand as a cell adhesion substrate, the contact time does not have a particular upper limit.

In another embodiment of the present invention, the treatment for activating the ITGB1-β-catenin signal transduction pathway includes, for example, bringing an accelerator of ITGB1 expression (e.g., extract of fucoid, rosemary, kiwi, Poria sclerotium, burdock, carrot or Rhodophyta (JP-A-H11-246428) etc.), a substance (e.g., antibody, antisense nucleic acid, siRNA or shRNA etc.) that inhibits expression or function of a substance that suppresses expression of ITGB1 (e.g., ZFYVE21, miR-29C, miR-124 etc.), a nucleic acid encoding ITGB1, or the like into contact with a pluripotent stem cell. These treatments can be performed by adding the above-mentioned substance to a medium for pluripotent stem cells. When the substance is a nucleic acid, a nucleic acid introduction reagent is preferably further added to promote introduction of the nucleic acid into the cell.

In another embodiment of the present invention, the treatment for activating the ITGB1-β-catenin signal transduction pathway includes a treatment to increase the expression of ILK in pluripotent stem cells and/or change the ILK into an activated state. Examples of such treatment include introducing a nucleic acid encoding ILK or a nucleic acid encoding a constitutive active form of ILK (e.g., membrane-localized constitutive active form obtained by adding myristoylated signal sequence to N-terminal of ILK or by adding CaaX motif to C-terminal) into pluripotent stem cells.

In still another embodiment of the present invention, the treatment for activating the ITGB1-β-catenin signal transduction pathway includes inhibiting GSK3β activity, particularly β-catenin phosphorylation activity, in pluripotent stem cells. GSK3β phosphorylates β-catenin and promotes homeostatic degradation by the ubiquitin proteasome system. Therefore, inhibition of GSK3β causes intracellular accumulation of β-catenin, resulting in activation of the Wnt/β-catenin signal transduction pathway.

Examples of the method of inhibiting GSK3β activity include a method of contacting pluripotent stem cells with a low-molecular-weight GSK3β inhibitor, a method of contacting pluripotent stem cells with a peptide that mimics a target phosphorylation site of β-catenin, and the like, and a method using a low-molecular-weight GSK3β inhibitor is preferable.

In the present invention, the GSK-3β inhibitor is defined as a substance that inhibits kinase activity of GSK-3β protein (e.g., phosphorylation capacity against β catenin), and many are already known. Examples thereof include lithium chloride (LiCl) first discovered as a GSK-3β inhibitor, BIO, which is an indirubin derivative (alias, GSK-3β inhibitor IX; 6-bromo indirubin 3'-oxime), SB216763 which is a maleimide derivative (3-(2,4-dichlorophenyl)-4-(1-methyl-1H-indol-3-yl)-1H-pyrrole-2,5-dione), GSK-3β inhibitor VII which is a phenyl α-bromomethyl ketone compound (4-dibromoacetophenone), L803-mts which is a cell membrane-permeable type-phosphorylated peptide (alias, GSK-3β peptide inhibitor; Myr-N-GKEAPPAPPQSpP-NH₂) and CHIR99021 having high selectivity (6-[2-[4-(2,4-Dichlorophenyl)-5-(4-methyl-1H-imidazol-2-yl)pyrimidin-2-ylamino]ethylamino]pyridine-3-carbonitrile). These compounds are commercially available from, for example, Calbiochem, Biomol and the like. The GSK-3β inhibitor may preferably be CHIR99021.

Pluripotent stem cells can be contacted with a GSK3β inhibitor by culturing the pluripotent stem cells in a medium added with the GSK3β inhibitor. The concentration of the GSK3β inhibitor to be added to the medium varies depending on the kind of the medicament. For example, when it is CHIR99021, the concentration is, for example, 1 µM, 2 µM, 3 µM, 4 µM, 5 µM, 6 µM, 7 µM, 8 µM, 9 µM, 10 µM, 15 µM, 20 µM, 25 µM, 30 µM, 35 µM, 40 µM, 45 µM, 50 µM or the like, though it is not limited to these. Preferably, it is not less than 5 µM (e.g., 5 µM - 50 µM, preferably 5 µM - 10 µM).

Pluripotent stem cells can be contacted with the GSK3β inhibitor for 2 - 5 days, preferably 3 - 4 days.

### (II) Culture kit for releasing resistance of pluripotent stem cell to differentiation into a mesendodermal lineage

The present invention also provides a reagent for releasing resistance of pluripotent stem cell to differentiation into a mesendodermal lineage, containing any of the aforementioned substances that activate the ITGB1-β-catenin signal transduction pathway. In one preferred embodiment, the substance that activates ITGB1-β-catenin signal transduction pathway is an ITGB1 binding ligand at a concentration capable of causing sufficient ITGB1 expression in pluripotent stem cells. The binding ligand is preferably provided in the form of a culture vessel coated with the same. Therefore, the present invention also provides a culture kit for releasing resistance of a pluripotent stem cell to differentiation into a mesendodermal lineage, containing the culture vessel as a constitution (hereinafter to be also referred to as "the kit of the present invention").

As a culture vessel coated with a binding ligand which is included in the kit of the present invention, for example, when the binding ligand is laminin 511 or a fragment thereof, a culture vessel coated with laminin 511 or a fragment thereof at a concentration corresponding to 0.005 - 0.25 µg/cm² (1/100 - 1/2 of recommended concentration 0.5 µg/cm²), preferably 0.025 - 0.125 µg/cm² (1/20 - 1/4 of recommended concentration), more preferably 0.03 - 0.07 µg/cm² (about 1/16 - 1/7 of recommended concentration), based on laminin 511 E8 fragment can be mentioned. As the culture vessel, those mentioned above as culture vessels for maintenance culture of pluripotent stem cells can be used in the same manner.

The kit of the present invention can further include a medium, a medium additive, a reagent for dissociation of cells from a culture vessel for transferring the cells to subculture or differentiation-inducing culture, a reagent for dissociation of cell-cell adhesion, and the like.

### (III) Method for inducing differentiation into cells of mesodermal lineage or endodermal lineage

The present invention also provides a method for producing a cell of a mesodermal lineage or endodermal lineage, comprising a step of inducing differentiation of a pluripotent stem cell released from resistance to differentiation into a mesendodermal lineage, which is produced by the above-mentioned production method (I) of the present invention, into a cell of a mesodermal lineage or endodermal lineage (hereinafter to be also referred to as "the production method (II) of the present invention"). A pluripotent stem cell produced by the production method (I) of the present invention is released from resistance to differentiation into a mesendodermal lineage, and thus can be efficiently induced to differentiate into a cell of a mesodermal lineage or an endodermal lineage.

The cell of a mesodermal lineage that may be produced by the production method (II) of the present invention may be any cell belonging to a mesodermal lineage as long as a differentiation induction system from a pluripotent stem cell is established., For example, blood cell, vascular endothelial cell, skeletal muscle cell, chondrocyte, kidney cell, cardiomyocyte, adipocyte and the like can be mentioned. The "blood cell" here means any cell differentiated from hematopoietic stem cell and contained in blood, and includes red blood cell, platelet, neutrophil, eosinophil, basophil, macrophage, NK cell, dendritic cell, T cell, B cell, progenitor cells thereof, and the like.

The cell of an endodermal lineage that may be produced by the production method (II) of the present invention may be any cell belonging to an endodermal lineage as long as a differentiation induction system from a pluripotent stem cell is established. For example, cells such as pancreatic β cell, hepatocyte, intestinal cell, lung cell, thyroid gland cell and the like, progenitor cells thereof, and the like can be mentioned.

The mesodermal lineage and the endodermal lineage use mesendodermal cell as a common precursor. Therefore, differentiation into mesendodermal cell is induced in the early stage of the step of inducing differentiation into a cell of a mesodermal lineage or an endodermal lineage. Differentiation into mesendodermal cell can be confirmed by examining the expression of mesendodermal markers such as T, Foxa2, Gsc, Mixl1 and the like.

Methods for inducing differentiation of pluripotent stem cells into any of the above-mentioned mesodermal lineage cells and endodermal lineage cells are well known in the art. For example, Nakatsuji and Suemori ed., "Experimental Medicine Separate Volume ES/iPS Cell Experimental Standard" (published by YODOSHA CO., LTD., 2014) describes methods for inducing differentiation into various mesodermal lineage cells or endodermal cells, and those of ordinary skill in the art can induce differentiation of pluripotent stem cells into various mesodermal lineage cells or endodermal lineage cells by referring to these books.

The pluripotent stem cells obtained by the production method (I) of the present invention are particularly superior in the efficiency of differentiation into blood cells. Therefore, in one preferred embodiment, the mesodermal lineage cells produced by the method (II) of the present invention are blood cells. As a method for inducing differentiation of pluripotent stem cells into hematopoietic progenitor cells, for example, the PSC-Sac method, which is a co-culture method with a mouse stromal cell C3H10TR, can be mentioned (Takayama and Eto Methods Mol Biol. 2012; 788:205-17, Takayama et al. J Exp Med. 2010 207(13): 2817-2830). This method is simple because the necessary cytokine is vascular endothelial growth factor (VEGF) only, and when maintenance-cultured pluripotent stem cells are detached from the substrate and seeded on mitomycin C (MMC)-treated C3H 10T1/2 cells, adhesion of the pluripotent stem cells to the feeder cells is observed on day 4, and when the culture is continued until day 14, Sac-like structures are formed from the pluripotent stem cells. Hematopoietic progenitor cells are obtained from the Sac-like structures.

The hematopoietic progenitor cells obtained as described above can be further differentiated into various blood cells by a method known per se. For example, methods for inducing differentiation into megakaryocyte progenitor cells, and further into platelets, are described in detail in WO 2018/038242.

### (IV) Evaluation method of resistance of pluripotent stem cell to differentiation into mesendodermal lineage

As mentioned above, pluripotent stem cells are released from resistance to differentiation into a mesendodermal lineage by activating the ITGB1-β-catenin signal transduction pathway. That is, expression of ITGB1 is promoted in pluripotent stem cells released from differentiation resistance. Therefore, the degree of resistance of the pluripotent stem cells to differentiation into a mesendodermal lineage can be evaluated using the expression of ITGB1 in the pluripotent stem cell as an index. That is, the present invention also provides a method for evaluating resistance of a pluripotent stem cell to differentiation into a mesendodermal lineage, comprising measuring an expression level of ITGB1 in the pluripotent stem cell (hereinafter to be also referred to as "the evaluation method of the present invention").

The pluripotent stem cell to be subjected to the evaluation method of the present invention has unknown resistance to differentiation into a mesendodermal lineage. The expression level of ITGB1 can be measured by, for example, contacting a test pluripotent stem cell with a fluoresce-labeled anti-ITGB1 antibody to visualize ITGB1-expressing cells, and performing image analysis using flow cytometry or a fluorescence microscope. The obtained ITGB1 expression level is compared with the control expression level (e.g., cutoff value set from the ITGB1 expression level in a considerable number of pluripotent stem cell clones known to have been released from differentiation resistance). When the expression level is not less than the control expression level, it can be determined that the test pluripotent stem cell has been released from resistance to differentiation into a mesendodermal lineage.

In another embodiment, a pluripotent stem cell induced from a cell that expresses a reporter gene (e.g., fluorescent protein gene) under the control of an ITGB1 gene promoter is used as the pluripotent stem cell, the pluripotent stem cell is subjected to maintenance culture in a culture vessel coated with a cell adhesion substrate, with regard to which a concentration that can achieve an ITGB1 expression level sufficient for activating the ITGB1-β-catenin signal transduction pathway is unidentified, and the above-mentioned evaluation method is conducted, whereby the concentration of the cell adhesion substrate that can achieve an ITGB1 expression level sufficient for activating the ITGB1-β-catenin signal transduction pathway can be determined. Therefore, the present invention also provides a method for producing a pluripotent stem cell released from resistance to differentiation into mesendodermal lineage, including contacting the pluripotent stem cell with the cell adhesion substrate at a concentration determined as described.

The present invention is further explained in the following by referring to Examples; however, the present invention is not limited by the Examples below.

### [Example]

### (material and method)

### 1. Pluripotent stem cell line

Human iPS cell line AK5 (Nakagawa et al., Scientific Reports, 4: 3954, 2014) used for differentiation experiment, imaging, Western blotting, flow cytometry, intracellular staining, and cell adhesion assay was distributed in the Center for iPS Cell Research and Application, Kyoto University. In addition, human iPS cell line 692D2 (Okita et al., Stem Cells, 31: 458-466 (2013)) used in Example 8 was distributed by Dr. Keisuke Okita, Center for iPS Cell Research and Application, Kyoto University, and human ES cell line KthES11S was distributed by Dr. Hirofumi Suemori, Institute for Frontier Life and Medical Sciences, Kyoto University.

### 2. Maintenance culture of pluripotent stem cells (PSCs)

### 2-1. Maintenance culture on mouse fetal fibroblast (MEF) feeder

PSCs were cultured according to the previous report on a 6 cm dish seeded with MEF treated with mitomycin C (Takayama et al., Blood, 111(11): 5298-5306, 2008). As the medium, Dulbecco modified Eagle medium/Nutrient Mixture F-12 Ham (Sigma-Aldrich, St. Louis, MO, USA) supplemented with MEM non-essential amino acids, 0.1 mM 2-mercaptoethanol, 20% KnockOut (trade mark) Serum Replacement (Gibco/Thermo Fisher, Waltham, MA, USA), 5 ng/mL basic fibroblast growth factor (bFGF; Wako, Osaka, Japan) was used.

### 2-2. Maintenance culture on laminin

PSCs were cultured according to the previous report on various laminin E8 fragments under feeder-free conditions (Nakagawa et al., Scientific Reports, 4: 3954, 2014). Among the recombinant laminin E8 fragments used, LM511 E8 was iMatrix-511/iMatrix-511 silk (Nippi, Ibaraki, Japan), and other laminin E8 (LM421/121) fragments were provided by Dr. Kiyotoshi Sekiguchi (Institute for Protein Research, Osaka University). Each laminin fragment was added to 0.5 µg/cm² relative to the dish bottom area. In the laminin dilution experiment, coating was performed in the same manner as described above under the conditions of [x1/2], [x1/4], [x1/8], [x1/16], wherein [x1] is Laminin 511E8 0.5 µg/cm² condition.

To be specific, the recombinant laminin E8 fragment was suspended in PBS(-), added onto a dish, and allowed to stand at 37°C for 1 hr or longer. A small amount of AK02N medium (Ajinomoto) was added, the mixture was conditioned, the laminin-PBS solution was removed by aspiration, and the medium was substituted with AK02N medium containing Y-27632. PSCs were seeded on these dishes. From the next day onward, the medium was exchanged with AK02N medium supplemented with Y-27632 as appropriate while observing the state of the cells, and the culture was continued.

### 3. Cell differentiation experiment

As a method for performing blood cell differentiation from PSCs, the PSC-Sac method, which is a co-culture method with mouse interstitial cell C3H10TR, was used (Takayama and Eto Methods Mol Biol. 2012; 788:205-17, Takaayama et al. J Exp Med. 2010 207(13): 2817-2830). The same number of PSCs were cultured in two wells, one well was washed with PBS, exposed with TrypLE (trade mark) Select (Thermo Fisher Scientific, Waltham, MA), and stood at 37°C for 4 min. After confirmation of the absence of intercellular adhesion, the cells were detached from the dish with Cell scraper, and the number of the cells was counted with Trypan Blue and a hemocytometer.

The other well was exposed to Dispase I (Wako, Osaka, Japan), stood at 37°C for 2 min, and the cells were detached with Cell scraper and gently suspended in a medium. PSCs were seeded to 0.7-1x10^5 cells on C3H10TR (Riken Bio-Resource Center, Tsukuba, Ibaraki, Japan) feeder cells treated with mitomycin C and seeded on a gelatin-coated 10 cm dish, stood overnight in 37°C, 5% CO₂ environment, and differentiation induction was started. On days 0-7, the cells were cultured in 5% O₂ environment. After the start of the differentiation induction, the medium was exchanged every 3-4 days with hematopoietic cell differentiation medium [IMDM media (Sigma-Aldrich, St. Louis, MO, USA) with 15% fetal bovine serum (FBS), insulin/transferrin/selenite solution (Gibco/Thermo Fisher, Waltham, MA, USA), Ascorbic acid, 1-thioglycerol (Sigma-Aldrich, St. Louis, MO, USA -Aldrich), 20 ng/ml recombinant human VEGF (VEGF-A165; Wako, Osaka, Japan)], the cells on the dish were recovered on day 14, and the number of the cells was counted with Trypan Blue and a hemocytometer.

In CHIR99021 (ADOOQ Bioscience, Irvine, CA, USA) addition experiment, CHIR99021 6 nM was added from 4 days, 3 days, 2 days, or 1 day, respectively, before the start of differentiation induction to the start of the differentiation induction, to the PSCs to be differentiated which were cultured and maintained on laminin 511 E8 fragment (LM511). In the group without addition of CHIR99021, the same amount of DMSO was added on the same day.

### 4. Flow cytometry (FCM)

The number of the cells was counted with Trypan Blue and a hemocytometer. Each sample was suspended in PBS (Staining medium) containing 3% FBS, various antibodies were added, and the sample was allowed to stand in a dark place on ice for 30 min and measured by FACS Verse (BD bioscience). For the measurement of hPSCs, TRA-1-60 positive cells were measured. As the antibody, Brilliant Violet 421 (trade mark) anti-human CD34 antibody (561), Pacific Blue (trade mark) Anti-human CD326 antibody (BioLegend, San Diego, CA, USA), APC conjugated CD43 monoclonal antibody (eBio84-3C1) (eBioscience/Thermo Fisher, Waltham, MA, USA), PE mouse anti-human CD29 antibody (BD, Bedford, MA, USA), Propidium Iodide (Sigma), Alexa Fluor (registered trade mark) 647 Mouse anti-Human TRA-1-60 Antigen (BD, Bedford, MA, USA) were used.

### 5. Imaging

Fluorescence micrographs were obtained with a high-speed multiphoton confocal laser microscope system (Nikon A1R system). Briefly, iPS cells were seeded on µ-Dish 35 mm (ibidi, GmbH, Martinsried, Germany) coated with various laminin fragments and cultured for 4 days. When colony formation was observed, the cells were fixed with 4% PFA for 15 min, washed, and then BB515 Mouse Anti-human CD29 (BD bioscience, Bedford, MA, USA) diluted with PBS containing 3% FBS was added, and the mixture was stood for 30 min. After thorough washing, observation was performed. Images were analyzed by NIS-Elements software (Nikon).

### 6. Western blotting

Western blotting was performed as described earlier (Eto et al., 2007; Nishikii et al., 2008, Nakamura et al., 2014). Briefly, a cell lysate sample (15 µl) obtained by treating the same number of cells was separated by electrophoresis on 4-15% Mini-PROTEAN (registered trade mark) TGX Precast Gels (Bio-Rad Laboratories, Hercules, CA) with a TNE buffer (10 mM Tris-HCl, pH 7.8, 150 mM NaCl, 1% NP-40 and 1 mM EDTA) added with a protease inhibitor cocktail (Roche, Basel, Switzerland) and a phosphatase inhibitor cocktail (Nacalai tesque, Kyoto, Japan). Then, transfer onto polyvinylidene difluoride membranes (GE Healthcare Life Sciences, Buckinghamshire, UK) were performed. A primary antibody reaction was performed using ILK1 (4G9), phospho-GSK3β (Ser9) (D85E12), β-catenin (D10A8) (CST, Chicago, USA), β-actin (AC-74) (Sigma-Aldrich, St. Louis, MO, USA), and visualized with SuperSignal (trade mark) West Pico Chemiluminescent Substrate (Thermo Fisher Scientific, Waltham, MA) using anti-mouse IgG-HRP (GE Healthcare, Little Chalfont, UK) and Anti-rabbit IgG, HRP-linked Antibody (CST, Chicago, USA) as the secondary antibodies.

### 7. Intracellular staining (ICS) and flow cytometry (FCM) analysis

To detect pluripotency markers SOX2 and OCT3/4, intracellular staining was performed by flow cytometry. Briefly, dead cell staining was performed using Zombie (trade mark) Aqua Fixable Viability Kit (BioLegend, San Diego, CA, USA). After washing, the cells were fixed by allowing to stand for 15 min at room temperature with PerFix-nckit Reagent1. Successively, the cells were subjected to a permeation treatment by fixing with ice-cooled 100% methanol for 5 min, and incubating for 10 min at room temperature with 0.5% saponin. The cells were washed and suspended in PBS (containing 3% FBS), V450 Mouse anti-Sox2 and Alexa Fluor (registered trade mark) 488 conjugated Mouse anti-OCT3/4 (BD biosciences, Bedford, MA, USA) was added, and the mixture was incubated on ice for 30 min. Excessive antibody was washed away and analysis was performed with FACSVerse (trade mark) (BD Biosciences).

### 8. Cell adhesion assay

By reference to Stem Cell Reports, Vol. 11. Issue 1, 142-156, 2018, the assay was performed with partial modification. A 48 well plate was coated with various laminins at 0.5 µg/cm², stood at 37°C for 1 hr or longer, and the medium was substituted with AK02N medium (Ajinomoto, Japan) supplemented with Y-27632. Passage-cultured human iPS cells were washed with PBS and exposed to TrypLE (trade mark) Select (Thermo Fisher Scientific, Waltham, MA) at 37°C for 10 min. Single cells were obtained by pipetting, seeded in each well by 0.5x10^5, and stood at 37°C, 5% CO₂ for 1 hr. Non-adherent cells were removed by washing twice with DMEM/F12 (Sigma-Aldrich, St. Louis, MO, USA). 4% PFA (Wako, Osaka, Japan) was added, the cells were fixed by allowing to stand for 15 min, 100% ethanol was added and the mixture was stood for 5 min. After substitution with methanol added with 0.4% Crystal Violet, the cells were stood at room temperature for 5 min. The cells were washed three times with desalted water and the supernatant was removed. The cells were washed with 250 µl of 1% SDS, and the optical density of the supernatant was measured by NanoDrop 2000c (Thermo Fisher Scientific, Waltham, MA) at 595 nm wavelength.

### (results)

### Reference Example 1 PSCs maintenance-cultured on laminin 511 E8 fragment (LM511) show differentiation resistance to hematopoietic cells

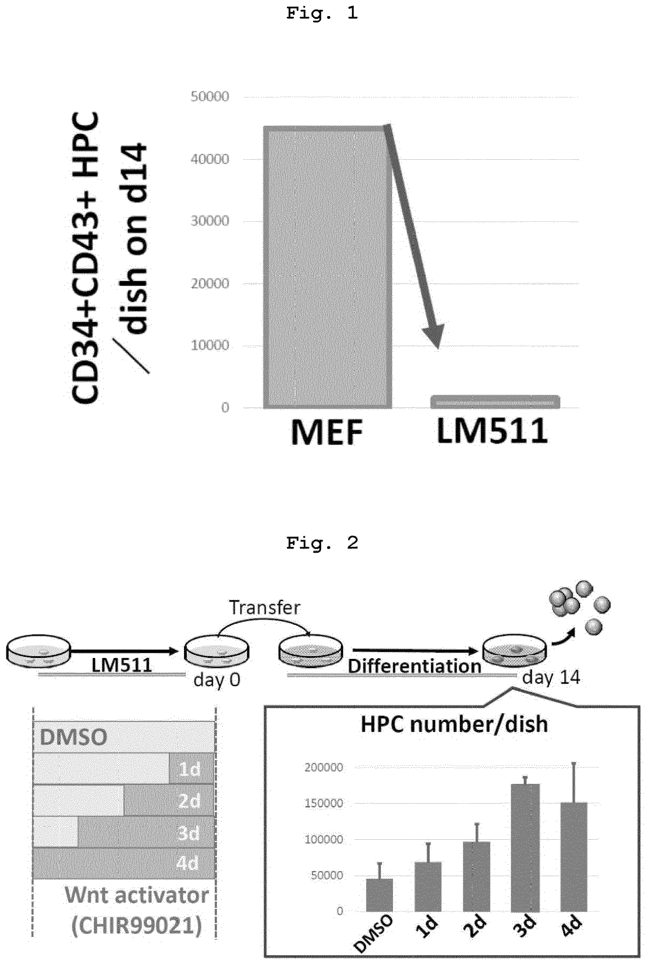

PSCs maintenance-cultured on MEF were detached from the substrate and seeded on MMC-treated C3H10TR cells. PSCs were found to have adhered to feeder cells on day 4, and when the culture was continued until day 14, a Sac-like structure was formed from PSCs. Hematopoietic progenitor cells were obtained in this Sac-like structure. On the other hand, when PSCs cultured and maintained in LM511 were similarly seeded into C3H10TR cells, they hardly adhered to the feeder cells, and a Sac-like structure was hardly observed even on day 14. The cells were recovered on day 14 and the hematopoietic progenitor cells of CD34⁺CD43⁺ were counted. As a result, almost no hematopoietic progenitor cells were obtained from the pluripotent stem cells cultured and maintained in LM511, reflecting the aforementioned observation (Fig. 1).

### Example 1 Wnt signal agonist releases resistance of PSCs cultured and maintained on LM511 to differentiation into hematopoietic cells

The present inventors previously reported that pluripotent stem cells maintained on laminin 511 showed resistance to differentiation into a mesendodermal lineage, whereas pluripotent stem cells maintained on laminin 421 and laminin 121 were released from this differentiation resistance and tended to easily differentiate into a mesendodermal lineage, particularly blood cells, and that pluripotent stem cells released from differentiation resistance showed an increase in the expression of the genes at the downstream of the Wnt/β-catenin transduction signal pathway (WO 2018/0378242). Therefore, whether the resistance to differentiation into hematopoietic cells was released by stimulating the Wnt/β-catenin signal transduction pathway in cells cultured and maintained on LM511, which could not produce hematopoietic progenitor cells, was examined. Accumulation of β-catenin in the cell was promoted by inhibiting GSK3β, which is an intermediate mediator of the signal transduction pathway, using CHIR99021. Using the cells treated with the GSK3β inhibitor, differentiation induction was performed by the PSC-sac method. As a result, induction of differentiation of PSCs, which were cultured and maintained on LM511, into hematopoietic cells was improved, and the number of hematopoietic progenitor cells could be increased (Fig. 2).

From these results, it was considered that the cell anchorage by laminin regulates the pathway to hematopoietic differentiation inside PSCs via the Wnt/β-catenin signal transduction pathway.

### Example 2 PSCs cultured and maintained on laminin having hematopoietic cell differentiation induction potential highly express integrin β1

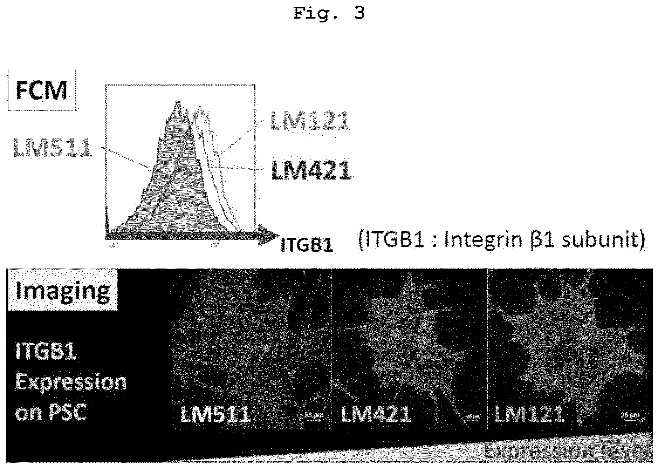

The expression level of integrin β1 (ITGB1) in PSCs cultured and maintained in each of LM511/LM421/LM121 was examined. ITGB1 is a cell surface protein known as a laminin receptor and has been reported to be important for cell survival and maintenance in PSCs. In observations using FCM and fluorescence microscope, PSCs cultured and maintained on LM421 and LM121 having hematopoietic characteristics showed higher expression of ITGB1 than LM511 having non-hematopoietic characteristics (Fig. 3).

### Example 3 Laminin having hematopoietic cell differentiation induction potential activates ITGB1-β-catenin signaling

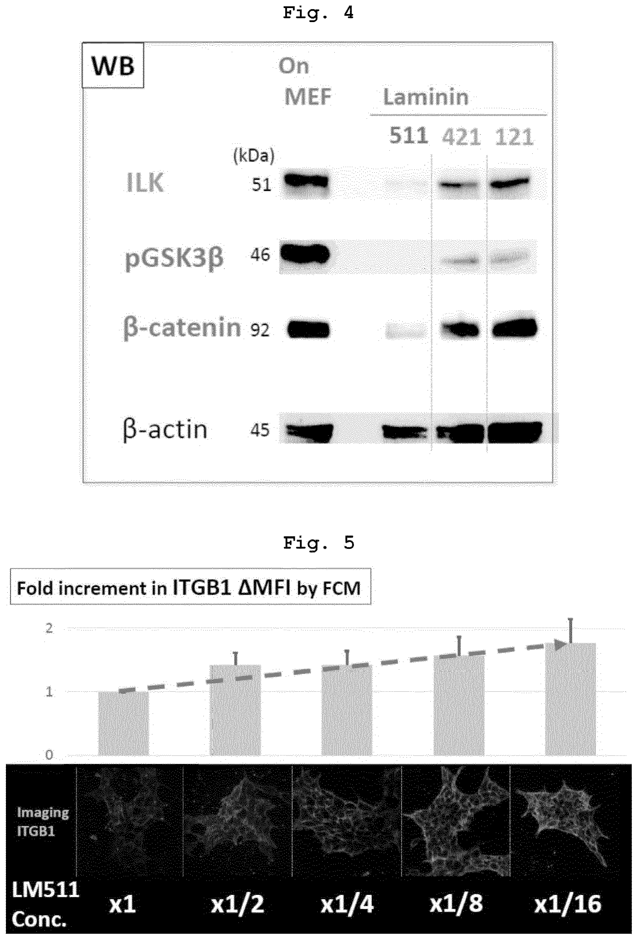

In some reports relating to cancer, ITGB1 is considered to change the accumulation amount of β-catenin via ILK/GSK3β and regulate the Wnt/β-catenin signal transduction pathway. Thus, to confirm whether the same pathway works in the relationship between laminin and PSCs, Western blotting analysis was performed. As a result, those maintained on MEF showed the highest amount of ILK/pGSK3β/β-catenin. In addition, those cultured and maintained on LM421 and LM121 showed a higher amount of ILK/pGSK3β/β-catenin than those cultured and maintained on LM511 (Fig. 4). These results suggest that an increase in the amount of ITGB1 on the cell surface made the signal below ITGB1 stronger, and increased the amount of β-catenin accumulated in the cell, thus resulting in the activation of the Wnt/β-catenin signal transduction pathway.

### Example 4 Relationship between LM511 concentration and ITGB1 expression

In the process of maintenance culture research of PSCs, the present inventors noticed that LM421/LM121 tends to show weaker cell-substrate adhesion than LM511. Therefore, whether the above-mentioned results are attributable to the kind of laminin or the "adhesion strength" between ITGB1 and laminin was confirmed. In this Example, PSCs were seeded on a plate coated with serially diluted LM511, which is a non-hematopoietic laminin, and cultured and maintained. By diluting LM511, the strength of adhesion (= number of integrin-laminin bonds) was adjusted without changing the laminin subunit of 5-1-1. As a result, it was shown in the analyses by FCM and fluorescence microscopy that the expression of ITGB1 is higher under weaker cell-substrate adhesion conditions, and the expression level of ITGB1 is dependent on the amount of cell-substrate adhesion, that is, the strength of adhesion, rather than the kind of the laminin subunit (Fig. 5).

### Example 5 PSCs cultured and maintained on diluted LM511 are efficiently induced into hematopoietic progenitor cells

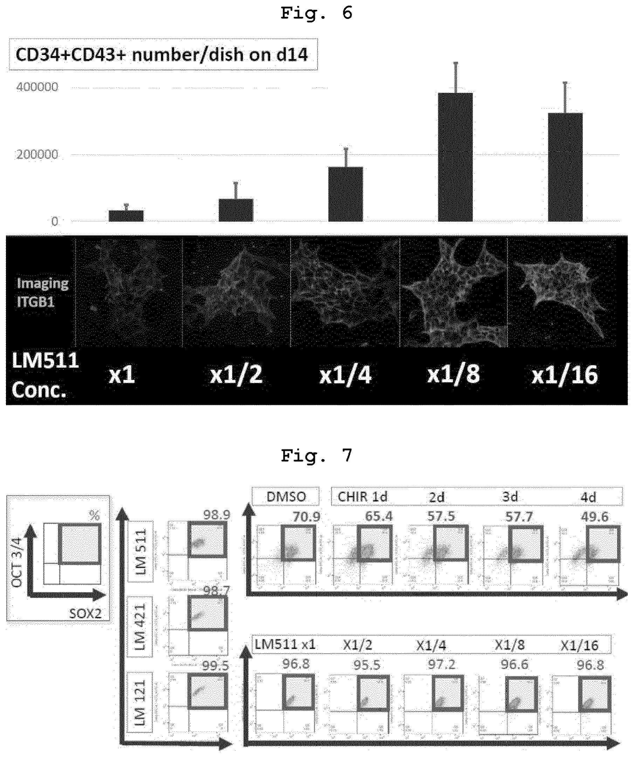

Next, PSCs cultured and maintained on the serially diluted LM511 were cultured by the PSC-sac method. As a result, the number of hematopoietic progenitor cells differentiated from the PSCs cultured and maintained on diluted LM511 was higher than the number of hematopoietic progenitor cells differentiated from the PSCs cultured and maintained at the recommended concentration (x1) of LM511, and the cells could be released from resistance to differentiation into blood cells caused by maintaining the culture in LM511 (x1) (Fig. 6). In addition, PSCs with a high expression level of ITGB1 showed higher hematopoietic capacity when differentiated into blood cells (Fig. 6).

Summarizing the above results, weak adhesion between extracellular substrate and cells induces expression of ITGB1, when a sufficient amount of ITGB1 is expressed, the downstream signal changes, β-catenin is accumulated to activate Wnt/β-catenin signal transduction pathway, and the differentiation of hematopoietic progenitor cells is promoted.

### Example 6 Regulation with GSK3β inhibitor prevents maintenance of pluripotency of PSCs

In Example 1, PSCs cultured and maintained in LM511 could be released from resistance to differentiation into blood cells by adding CHIR99021. The number of cells maintaining positive pluripotency for both OCT3/4 and SOX2 in the PSCs decreased immediately before initiation of differentiation by seeding on C3H10TR feeder cells. In contrast, the proportion of cells maintaining positive pluripotency for both OCT3/4 and SOX2 is high when a method including serially diluting LM511 is used or when other laminin such as LM421/121 and the like is used for maintenance, and both "maintenance of pluripotency" and "release of differentiation resistance" could be achieved (Fig. 7) .

### Example 7 Cell-substrate adhesion strength of various laminins

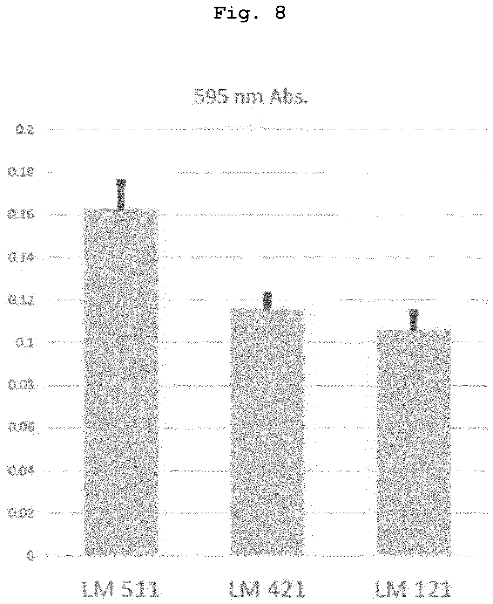

The cell-substrate adhesion strength was compared between the hematopoietic LM421 and LM121, and the non-hematopoietic LM511. As a result, it was confirmed that hematopoietic LM421 and LM121 had weaker cell-substrate adhesion than non-hematopoietic LM511 (Fig. 8).

### Example 8 Laminin 511 concentration that can release differentiation resistance is different for each pluripotent stem cell clone

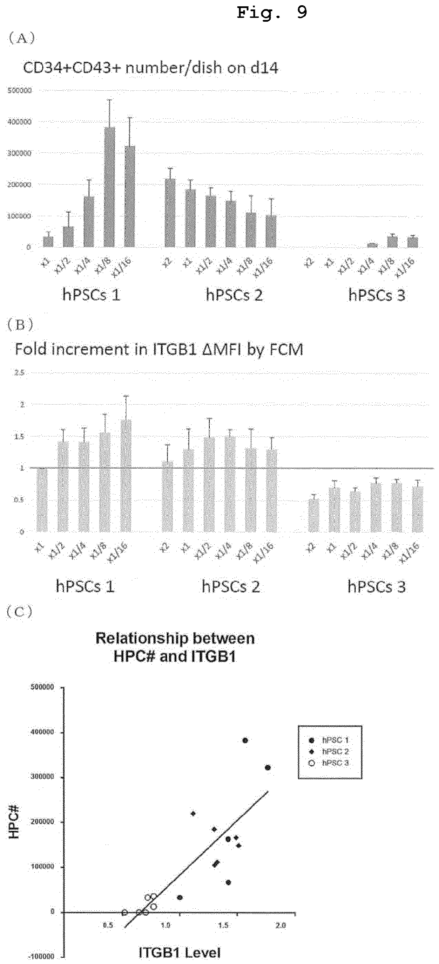

Human iPS cell line AK5 (hPSC1), human iPS cell line 692D2 (hPSC2) and human ES cell line KthES11 (hPSC3) were examined for the relationship between LM511 concentration and ITGB1 expression, and the relationship between LM511 concentration and differentiation potential into hematopoietic progenitor cell in the same manner as in Examples 4 and 5. As a result, in hPSC1, the expression level of ITGB1 was higher and the differentiation efficiency into hematopoietic progenitor cells was higher when the maintenance culture was performed with the diluted LM511 than when the maintenance culture was performed with the recommended concentration (x1) of LM511. In hPSC1, sufficient ITGB1 expression level was achieved even when maintenance-cultured on the recommended concentration (x1) of LM511, and resistance to differentiation into hematopoietic progenitor cells was not observed. The ITGB1 expression level did not change significantly even when LM511 was serially diluted, and the differentiation efficiency into hematopoietic progenitor cells was found to be rather high at the recommended concentration or two-fold concentration (Figs. 9A and B). hPSC3 hardly differentiated into hematopoietic progenitor cells at the recommended concentration of LM511 and showed high differentiation resistance. However, a certain degree of differentiation potential into hematopoietic progenitor cells was obtained by using serially diluted LM511 (Fig. 9A and B). A positive correlation was found between the ITGB1 relative expression level of PSCs and the efficiency of differentiation into hematopoietic progenitor cells (Fig. 9C).

From the above results, it was shown that the ease of differentiation of pluripotent stem cell clone into a mesendodermal lineage can be evaluated using the expression level of ITGB1 as an index and, as for the pluripotent stem cells judged to have differentiation resistance at the recommended concentration of LM511, they can be released from the differentiation resistance by using LM511 diluted to a concentration lower than the recommended concentration.

### [Industrial Applicability]

According to the present invention, since pluripotent stem cells are released from resistance to differentiation into a mesendodermal lineage by diluting laminin 511 or a fragment thereof to a concentration lower than the recommended concentration, the cost reduction of maintenance culture and efficient differentiation induction can be simultaneously achieved. Thus, the present invention is extremely useful in the production of a cell of a mesendodermal lineage.

This application is based on a patent application No. 2018-205912 filed in Japan (filing date: October 31, 2018), the contents of which are incorporated in full herein by reference.

## Claims

1. A method for producing a pluripotent stem cell released from resistance to differentiation into mesendodermal lineage, comprising subjecting a pluripotent stem cell to a treatment for activating an integrin β1-β-catenin signal transduction pathway (excluding contacting the pluripotent stem cell with laminin 421 or a fragment thereof and/or laminin 121 or a fragment thereof).

2. The method according to claim 1, wherein the pluripotent stem cell is further released from resistance to differentiation into a mesodermal lineage.

3. The method according to claim 2, wherein the pluripotent stem cell is further released from resistance to differentiation into a blood cell.

4. The method according to any one of claims 1 to 3, wherein the activation treatment is bringing the pluripotent stem cell into contact with laminin 511 or a fragment thereof corresponding to 0.005 - 0.25 µg/cm² of laminin 511 E8 fragment.

5. The method according to any one of claims 1 to 3, wherein the activation treatment is bringing the pluripotent stem cell into contact with laminin 511 or a fragment thereof corresponding to 0.03 - 0.07 µg/cm² of laminin 511 E8 fragment.

6. The method according to any one of claims 1 to 5, wherein not less than 90% of the pluripotent stem cells are Oct3/4-positive and Sox2-positive.

7. The method according to any one of claims 1 to 3, wherein the activation treatment is contacting the pluripotent stem cell with a GSK3β inhibitor for 2 - 5 days.

8. The method according to any one of claims 1 to 7, wherein the pluripotent stem cell is derived from human.

9. A method for producing a cell of a mesodermal lineage or endodermal lineage, comprising
(1) a step of producing a pluripotent stem cell released from resistance to differentiation into a mesendodermal lineage by the method according to any one of claims 1 to 8, and
(2) a step of inducing differentiation of the cell obtained in step (1) into a cell of a mesodermal lineage or endodermal lineage.

10. The method according to claim 9, wherein the cell of a mesodermal lineage is a blood cell, a vascular endothelial cell, a skeletal muscle cell, a chondrocyte, a kidney cell, a cardiomyocyte or an adipocyte.

11. The method according to claim 9, wherein the cell of a mesodermal lineage is a blood cell.

12. The method according to claim 9, wherein the cell of an endodermal lineage is a pancreatic β cell, a hepatocyte, an intestinal cell, a lung cell or a thyroid gland cell.

13. A culture kit for releasing resistance of a pluripotent stem cell to differentiation into a mesendodermal lineage, comprising a culture container coated with laminin 511 or a fragment thereof corresponding to 0.005 - 0.25 µg/cm² of laminin 511 E8 fragment.

14. The kit according to claim 13, wherein the culture container is coated with laminin 511 or a fragment thereof corresponding to 0.03 - 0.07 µg/cm² of laminin 511 E8 fragment.

15. A method for evaluating resistance of a pluripotent stem cell to differentiation into a mesendodermal lineage, comprising measuring an expression level of integrin β1 in the pluripotent stem cell.
